# FASCICULE DE BREVET EUROPEEN

(11) **EP 1 284 975 B1**
(45) Date de publication et mention de la délivrance du brevet: **18.08.2004**
(21) Numéro de dépôt: 01938303.3
(22) Date de dépôt: 22.05.2001
(51) Int. Cl.: C07D 453/04, C07B 53/00

(54) **AMMONIUMS PORTEURS D'UN FLUOR ELECTROPHILE, REACTIF EN CONTENANT, PROCEDE LES METTANT EN OEUVRE ET PROCEDE DE SYNTHESE POUR LES OBTENIR**
AMMONIUMVERBINDUNGEN,ENTHALTEND EIN ELEKTROPHILES FLUR ,REAGENZ DAS DIESE VERBINDUNGEN ENTHÄLT UND EIN VERFAHREN ZU DEREN HERSTELLUNG UND VERWENDUNG
AMMONIUM COMPOUNDS BEARING AN ELECTROPHILIC FLUORINE, REAGENT CONTAINING SAME, METHOD USING SAME AND SYNTHESIS METHOD FOR OBTAINING THEM

(30) Priorité: 23.05.2000 FR 0006557
(43) Date de publication de la demande: 26.02.2003
(73) Titulaire: RHODIA CHIMIE, 92512 Boulogne Billancourt Cedex (FR)
(72) Inventeur: DESMURS, Jean-Roger, F-69360 Saint-Symphorien D'Ozon (FR); HEBRAULT, Dominique, 01945 Marblehead MA (US); CAHARD, Dominique, F-76960 Notre-Dame de bondeville (FR); AUDOUARD, Christophe, F-76120 Le Grand Quevilly (FR); PLAQUEVENT, Jean-Christophe, F-76960 Notre-Dame de Bondeville (FR)
(74) Mandataire: Ricalens, François
(86) Numéro de dépôt international: PCT/FR2001/001578
(87) Numéro de publication internationale: WO 2001/090107

(56) Documents cités:
- EP-A- 0 745 602
- PATENT ABSTRACTS OF JAPAN vol. 1998, no. 01, 30 janvier 1998 (1998-01-30) & JP 09 249653 A (YAKULT HONSHA CO LTD), 22 septembre 1997 (1997-09-22)
- POSS A J ET AL: "1-Fluoro-4-hydroxy-1,4-diazoniabicyclo[2. 2.2]octane Bis(tetrafluoroborate): An Electrophilic Fluorinating Agent" TETRAHEDRON LETTERS,NL,ELSEVIER SCIENCE PUBLISHERS, AMSTERDAM, vol. 40, no. 14, 2 avril 1999 (1999-04-02), pages 2673-2676, XP004159407 ISSN: 0040-4039
- DREW M D ET AL: "The Asymmetric Reduction Of Ketones Using Chiral Ammonium Fluoride Salts And Silanes" TETRAHEDRON LETTERS,NL,ELSEVIER SCIENCE PUBLISHERS, AMSTERDAM, vol. 38, no. 33, 18 août 1997 (1997-08-18), pages 5857-5860, XP004085893 ISSN: 0040-4039

## Description

La présente invention a pour objet de nouveaux agents de fluoration électrophile. Elle vise plus spécifiquement des composés à fonction ammonium quaternaire dont l'azote porte un atome de fluor et l'utilisation de ces molécules ammoniums quaternaires pour former un réactif susceptible de réaliser des fluorations électrophiles énantiosélectives (FEE).

Dans la suite de la description, nous nous conformerons à l'usage selon lequel on désigne les composés chimiques par une fonction importante considérée, fonction qui devient alors l'éponyme des composés considérés.

Ainsi, les composés porteurs de la fonction ammonium quaternaire visée dans la présente invention seront désignés par "ammonium quaternaire".

La fluoration des composés organiques a toujours été un problème délicat en raison des spécificités de l'atome et de la molécule de fluor. La molécule de fluor présente une telle réactivité que les fluorations directes sont quasiment impossibles, sauf pour quelques familles de composés bien particuliers.

Aussi s'adresse-t-on généralement par des voies indirectes, soit par le biais des fluorures cobaltiques, soit par chloration, ou plus généralement halogénation et échange chlore-fluor.

Le problème est encore plus aigu lorsque l'on désire obtenir des composés fluorés de l'atome porteur du fluor présentant une chiralité.

Le problème est d'autant plus aigu que l'on réalise l'introduction du fluor tard dans la synthèse du composé désiré.

Or, au cours des trois derniers lustres, les composés fluorés ont pris une importance grandissante dans les molécules à activité biologique, notamment pharmaceutique et agrochimique.

Aussi, le marché est demandeur de réactifs et d'agents de fluoration qui évitent de réaliser la fluoration en plusieurs étapes et qui ne soient pas d'un prix prohibitif.

Les fluorations électrophiles présentent l'avantage de ne nécessiter en général que peu d'étapes.

Cependant, il existe extrêmement peu de réactifs intéressants et ayant une large gamme d'efficacité dans le domaine de la fluoration énantiosélective.

Pour mémoire, on peut citer les travaux de E. Differding *Tetrahedron Letters*, 29, 1988, 6087-6090 qui a proposé d'utiliser des fluorosultames pour réaliser des fluorations énantiosélectives. Cette technique ne donne de bons résultats que pour peu de substrats et ne vise guère que les dérivés de type malonique. Dans les autres cas, les rendements et l'excès énantiomérique restent très faibles.

Ces résultats ont été un peu améliorés par F.A. Davis (J. Org. Chem., 63, 1998, 2273-2280), mais les sultames restent des composés difficiles d'accès et particulièrement coûteux.

Parallèlement à ces études, certains auteurs (voir l'article de R. Banks dans Journal of Fluorin Chemistry, 87 [1998] 1-17) ont montré que l'on pouvait fluorer certains dérivés de la DABCO (diazabicyclooctane) pour donner un des azotes quaternaires fluorés et qu'un tel réactif pouvait servir d'agent de fluoration dans certains cas.

A.S.Poss (Tetrahedron Letters, 40, 1999, 2673-2676) décrit une diamine bicyclique fluorée comme agent de fluoration achiral.

Toutefois, cette technique ne conduit pas à une fluoration énantiosélective.

Ces composés fluorés de la DABCO sont actuellement commercialisés sous la marque de fabrique Selectfluor.

C'est pourquoi un des buts de la présente invention est de fournir un nouvel agent de fluoration qui soit susceptible de donner de bons rendements de fluoration.

Un autre but de la présente invention est de fournir un agent de fluoration du type précédent qui soit susceptible de conduire à des fluorations énantiosélectives avec un excès énantiomérique significatif.

Un autre but de la présente invention est de fournir un réactif mettant en oeuvre un tel agent de fluoration, un autre but de la présente invention est de fournir un procédé de synthèse des agents de fluoration selon l'invention.

Un autre but, enfin, de la présente invention est de fournir un procédé d'utilisation du réactif de fluoration pour donner de bons rendements et un bon excès énantiomérique (ee).

Ces buts, et d'autres qui apparaîtront par la suite, sont atteints au moyen par un ammonium quaternaire présentant au moins un atome asymétrique et dont la fonction ammonium quaternaire est porteuse d'un fluor et n'est distante par le chemin le plus court que d'au plus quatre chaînons, avantageusement au plus trois chaînons, de préférence au plus deux chaînons, dudit atome asymétrique (le plus proche quand il y en a plus de 1). Par chaînons, on entend des atomes ou des groupes divalents dont le nombre des chaînons ne compte ni le carbone asymétrique considéré, ni l'azote quatemarisé par le fluor.

Ainsi, si l'on se place dans le produit exemplifié dans la présente demande, à savoir le N-fluorocinchonidinium, deux atomes asymétriques répondent à la condition 1 qui est directement liée à l'azote quaternaire et l'autre qui est lié à l'azote quaternaire par un chaînon, lequel chaînon est le carbone chiral précédent.

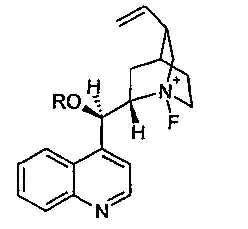

Sur cette formule, on s'aperçoit que l'on peut prendre plusieurs chemins pour aller de l'atome d'azote quaternaire aux divers atomes chiraux. Le chemin à prendre en compte est celui qui comporte le moins de chaînons.

La fonction alcool peut être protégée (éther, ester ...).

La formule générale des composé selon 'invention préférés peut s'ecrire

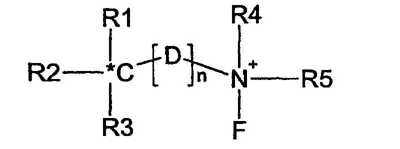

Où R₁, R₂, et R₃, sont différents et sont choisis parmi l'hydrogène, les halogènes, les fonctions ci après :
◆ -alcool ;
◆ amine ;
◆ amide ;
◆ thiol :
où R₄, et R₅, sont choisis parmi les dérivés hydrocarbonés
où D représentent les maillons divalents assurant la liaison entre le carbone chiral et l'ammonium quaternaire fluoré
où n reprsente un entier au plus égal à 4, avantageusement à 3, de préférence à 2. Et avantageusement au moins égal à 1.
les éventuels D, qui peuvent être identiques ou différents, sont
avantageusement choisis parmi les chalcogènes, les méthylènes éventuellement substitués, et les atomes métalloïdes de la colonne VB tel que l'azote, (La classification périodique des éléments utilisée dans la présente demande est celle du supplément au Bulletin de la Société Chimique de France, janvier 1966, n° 1).

Compte tenu de l'instabilité qu'ils conférerait à la molécules, il est souhaitable que lorsque D représente deux chalcogènes, surtout s'ils sont identiques les chalcogènes doivent être séparés par au moins un carbone, de préférence deux.

Il est préféré que les D soient tous choisis parmi les méthylènes, éventuellement substitués, et l'oxygène, pour au plus l'un d'entre eux. Il est préférable que R₁, soit un groupe de petite taille avantageusement méthyle ou mono atomique tel que Halogène et surtout hydrogène.

Avantageusement R₂ représente un radical hydrocarboné, c'est-à-dire contenant du carbone, et de l'hydrogène ou une fonction alcool, notamment R₂ peut être alcoyle, y compris aralcoyle, aryle, y compris alcoylaryle, alcoyloxyle, y compris aralcoyloxyle, aryloxyle, y compris alcoylaryloxyle. R₂ est aussi de préférence un radical issu de l'estérification d'un acide organique ou minéral par la fonction alcool, en particulier un radical acyloxyle. les estérifications par les acides aromatiques (c'est-à-dire les acides dont la fonction porteuse de l'acidité est directement reliée à un noyau aromatique tels que les acides benzoïques ou arène sulfonique), surtout les acides carboxyliques aromatiques donnent de très bons résultats.

R₃ est avantageusement un radical hydrocarboné, avantageusement aromatique souvent déprimé en électrons, tels les nitrobenzènes ou les noyaux pyridiniques, y compris quinoléiniques.

Dans le cas du N-fluorocinchonidinium les composés répondant à la formule :

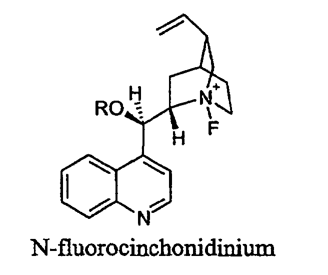

Comptent parmi les plus actifs.

Avec choisi parmi les aryles, les alcoyles, les acyles aromatiques et aliphatiques.

Selon la présente invention, les ammoniums sont de préférence tels que l'atome asymétrique, ou au moins l'un d'entre eux, est un atome de carbone.

Il est préférable que cet atome asymétrique soit porteur d'une fonction hydrogénophore non oxydable. Par hydrogénophore, il convient d'entendre porteur d'hydrogène et par non oxydable, il faut entendre que cette fonction n'est pas susceptible d'être oxydée par la fonction ammonium quaternaire porteuse d'un fluor.

Ces fonctions hydrogénophores peuvent notamment être des fonctions thiols, des fonctions amines, des fonctions amides ou des fonctions alcools. Toutefois, les fonctions thiols risquent d'être trop oxydables, les fonctions amines et les fonctions amides sont susceptibles d'interférer au cours de la réaction, surtout quand on utilise des bases; aussi préfère-t-on les fonctions alcools.

Les fonctioni alcool notamment acylées ou éthérifiées donne aussi de bon résultats.

La fonction ammonium quaternaire, outre l'atome de fluor, est avantageusement reliée à des atomes de carbone, avantageusement tous d'hybridation sp³.

Ainsi qu'on l'a vu plus haut, ledit atome asymétrique, ou l'un des atomes asymétriques, est porteur d'une fonction hydrogénophore susceptible de donner lieu à des liaisons hydrogènes et favorise ipso facto l'induction chirale. D'autres groupes peuvent aussi améliorer l'induction chirale, seuls ou en association avec ladite fonction hydrogénophore. Il s'agit en particulier de radicaux de groupes portant un noyau aromatique, souvent déprimé en électrons, tels les nitrobenzènes ou les noyaux pyridiniques, y compris quinoléiniques. Le dernier radical est avantageusement distinct des trois autres et est souvent méthyle, ou de préférence hydrogène.

Ainsi, selon une mise en oeuvre préférée de la présente invention, l'atome chiral considéré comporte comme substituant :
- un bras le reliant à la fonction ammonium quaternaire ;
- une fonction hydrogénophore ;
- un groupe porteur d'une fonction arylique, en général un aryle ou un aralcoyle, éventuellement substitué ;
- un atome ou un groupe avantageusement petit tel que l'hydrogène et le méthyle.

Selon une des mises en oeuvre préférées de la présente invention, surtout lorsque le carbone asymétrique considéré est porteur d'un hydrogène, il peut être intéressant de protéger la fonction hydrogénophore (alcool, amide, voire thiol et amine) ce qui réduit alors le risque d'oxydation en maintenant la possibilité d'une induction chirale forte comme radical assurant la protection, on peut citer les alcoyles, les acyles, les sulfonyles aromatiques ou aliphatiques et les groupes protecteurs usuellement connus dans la chimie des alcaloïdes, des peptides et des acides nucléiques. Les inductions chirales issues des alcools protégés par des alcoyles et par des acyles sont remarquables. Les alcoyles (pris dans le sens étymologique d'un alcool dont on a enlevé la fonction OH), y compris aralcoyles, présente avantageusement au plus 10 atomes de carbone et de préférence au plus 5 atomes, plus préféréntiellement 3.

Dans le cadre de la mise en oeuvre ci dessus de la présente invention, on peut exprimer les préférences sur le, ou l'un des, carbone asymétrique comme ci après. , l'atome chiral considéré comporte comme substituant
- un bras le reliant à la fonction ammonium quaternaire;
- une fonction hydrogénophore protégé, avantageusement une fonction alcool protégée sous la forme d'un éther ou d'un ester ;
- un groupe porteur d'une fonction arylique, en général un aryle ou un aralcoyle, éventuellement substitué ;
- un atome ou un groupe avantageusement petit tel que l'hydrogène et le méthyle.

La fonction ammonium quaternaire est avantageusement intracyclique, c'est-à-dire qu'elle constitue le chaînon d'un cycle, lequel cycle n'est avantageusement pas aromatique. Cette fonction est en général chirale.

Selon la présente invention, il est préféré que la fonction ammonium quaternaire constitue le chaînon d'au moins deux cycles.

Il est souhaitable que le contre-ion de l'ammonium quaternaire soit un anion peu polarisant et mauvais nucléophile.

Ces propriétés sont souvent corrélées à la force de l'acide associé à l'anion, aussi préfère-t-on des contre-ions dont l'acide associé présente un pKa au plus égal à deux, avantageusement à un ou de préférence à zéro.

On rencontre aussi ce type de contre-ions dans les anions comportant plusieurs atomes et donc que l'on peut qualifier de polyatomiques. En particulier, on peut citer les anions complexes à base de fluor tels que PF₄⁻, PF₆⁻. On peut également citer la première acidité de l'acide sulfurique, les chlorates et perchlorates, les acides organiques perfluorés sur le carbone portant la fonction acide, tels que les acides perfluoroalcanoïques et les acides sulfoniques porteurs d'un carbone perfluoré, comme par exemple l'acide triflique. Les imides correspondant à de tels acides perfluorés sur le carbone porteur de la fonction sulfonique donnent également de très bons résultats. Comme exemple de tels imines, on peut citer le triflimide.

D'une manière générale, les acides qui correspondent aux anions utilisés comme électrolytes dans les milieux non aqueux et dans les piles, notamment au lithium, donnent en général de bons résultats en tant que contre-ions des ammoniums quaternaires selon la présente invention.

Les anions préférés, ou du moins les plus utilisés, sont les triflates, les fluoborates et les ions PF₆⁻. On préfère les anions correspondant à des monoacides bien que les diacides, tels que par exemple l'acide fluosilicique, puissent être utilisés.

Ainsi que cela a été mentionné plus haut, un autre but de la présente invention est de fournir un réactif de fluoration électrophile utilisant les ammoniums selon la présente invention.

Ainsi, la présente invention vise un réactif comportant un ammonium tel que décrit ci-dessus, c'est-à-dire un réactif comportant un ammonium quaternaire présentant au moins un atome asymétrique et dont la fonction éponyme est porteuse d'un fluor et est située à quatre chaînons au plus dudit carbone asymétrique.

Selon la présente invention, ledit réactif comporte en outre un solvant avantageusement aprotique. Il est également préférable que ce solvant soit polaire.

Comme on le verra ultérieurement, compte tenu du fait que pour obtenir de bons excès énantiomériques il est souhaitable de travailler à basse température, il est avisé de choisir comme solvant ceux qui présentent un point de fusion très bas. Ainsi, il est préférable que le solvant présente un point de fusion au plus égal à 10°C, de préférence à -20°C, plus préférentiellement à -40°C.

Parmi les solvants donnant de bons résultats, on peut citer les éthers, notamment cycliques, les nitriles et même des dérivés aromatiques.

Les dérivés préférés sont les éthers cycliques et les nitriles.

Le réactif selon la présente invention comporte souvent pour addition successive ou simultanée, outre l'ammonium selon la présente invention et éventuellement le solvant, une base. Avantageusement, l'acide associé de la base présente un pKₐ d'au moins 16, de préférence d'au moins 18.

La nature de la base n'est pas indifférente au rendement lié à l'excès énantiomérique. Pour obtenir de bons rendements et de bons excès énantiomériques, il est souhaitable que le cation de la base ne soit pas trop petit. Il est ainsi préférable d'utiliser des cations alcalins de rang supérieur à celui du lithium. Les bases de type Schwesinger donnent aussi de bons résultats (cf. Tetrahedron Letters 39 [1998] 8775-8778).

On peut également utiliser en outre les métaux alcalins, les oniums (ammoniums quaternaires et phosphoniums tétraalcoylés). Selon la présente invention, il a été également montré qu'il était préférable que la base, ou plus exactement l'anion de la base, ne reste pas dans le milieu réactionnel et par exemple soit volatile.

Ainsi, il a été montré que les hydrures, qui après arrachement d'un proton, se dégagent sous forme d'hydrogène, donnent des résultats meilleurs que la plupart des autres bases.

Quoique non volatils, les sels alcalins de l'hexaméthyldisilazane donnent de bons résultats, notamment en excès énantiomérique.

La base peut également être réalisée in situ, par exemple par l'action d'un ion fluorure sur des dérivés silylés. La quantité de base peut avoir une certaine importance et le réactif peut en comprendre jusqu'à deux fois, voire plus, exprimés en équivalents dudit ammonium quaternaire.

L'excès de base améliore les rendements, mais ne modifie que peu les excès énantiomériques.

L'objet de la présente invention est également de fournir un procédé de synthèse des ammoniums fluorés selon la présente invention. Ces composés peuvent notamment être réalisés par l'action d'un réactif réputé agir comme "F⁺" mais selon un mode préféré de la présente invention, ces composés sont réalisés en mettant l'amine correspondante avec l'agent de fluoration désigné par Selectfluor et désigné souvent par le sigle F-TEDA-BF₄⁻. Cette réaction est avantageusement réalisée dans un solvant et à température ambiante. L'acétonitrile donne de bons résultats en tant que solvant.

Selon un mode préféré de la présente invention, on soumet l'amine qui, par fluoration, donnera l'ammonium selon la présente invention en quantité équimoléculaire avec ledit Selectfluor® dans un solvant, en général de l'acétonitrile. On laisse agiter de ¼ h à 5 h puis on évapore le solvant au moyen d'un évaporateur rotatif. On précipite l'amine issue du Selectfluor au moyen d'un acide fort (souvent l'acide sulfurique) après filtration et élimination du sel, on précipite au moyen d'un tiers solvant à partir d'une solution d'acétone. La filtration permet d'obtenir le composé désiré. Ce mode opératoire permet l'obtention des ammoniums selon la présente invention issus des alcaloïdes du quinquina, en particulier ont été ainsi obtenus, le N-fluorocinchonididium, le N-fluorocinchoninium, le N-fluoroquininium et le N-fluoroquinidinium. Les rendements obtenus sont excellents, surtout pour les deux premiers membres de la famille citée. Un autre but de la présente invention est de fournir un procédé de fluoration électrophile susceptible d'être stéréo-sélectif en utilisant les réactifs de la présente invention.

Selon la présente invention, ce but est atteint en mélangeant le réactif tel que défini ci-dessus avec le substrat et en laissant réagir pendant ¼ h à 24 h à des températures pouvant aller de -100°C à 50°C.

L'excès énantiomérique dépend de la température et il est souvent avisé de se placer à une température inférieure à -20°C, de préférence au plus égale à 30°C.

On peut se placer en particulier à -78°C, température qui est aisée à obtenir en utilisant de la carboglace.

Les substrats susceptibles de donner de bons résultats sont des molécules présentant un hydrogène mobile sur un carbone prochiral ou même chiral.

L'hydrogène est rendu mobile par la présence de groupes électro-attracteurs portés par le carbone chiral ou prochiral. On considère dans la présente description qu'un groupe électro-attracteur est un groupe présentant un σₚ positif et avantageusement supérieur à 0,5, plus préférentiellement supérieur à 0,10. Les meilleurs résultats sont obtenus avec des valeurs supérieures à 30. On ne comprend pas parmi ces groupes électro-attracteurs les atomes d'halogènes. Parmi les groupes attracteurs préférés, on peut notamment citer les groupes trifluorométhyle, et plus préférentiellement les groupes carbonyle et nitrile.

Les composés de nature malonique donnent des résultats acceptables.

Pour des raisons de rendement volumique, il est préférable que l'ammonium selon la présente invention présente moins de 50 atomes de carbone, de préférence au plus 30.

Il en va de même pour les substrats.

Une autre catégorie de substrats donne de bons résultats, il s'agit des doubles liaisons riches en électrons telles que, par exemple, les éthers d'énols ou les esters d'énols. Mention particulière doit être faite des éthers d'énols et de silyle.

Dans le cas des dérivés des énols, la base n'est pas toujours nécessaire.

Les exemples non limitatifs suivant illustrent l'invention.

### GENERALITES

Les appareils utilisés lors de toutes les analyses sont les suivant :
- Infra Rouge : spectrophotomètre IR-FT Perkin Elmer 16 PC.
- RMN ¹H(300 MHz), ¹³C(75 MHz), ¹⁹F(282 MHz).
- Polarimétrie : Polarimètre Perkin Elmer 241 PE.
- CPG sur colonne chirale (Supelco-β-dex).

### Exemple 1 - Synthèse des ammoniums selon la présente invention : synthèse de tétrafluoroborate de N-fluoroquininium

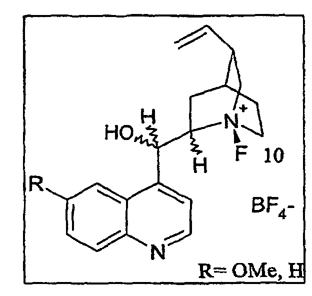

Dans un ballon bicol de 100 ml, on place l'alcaloïde (13,6 mmol) de départ en solution dans 50 ml d'acétonitrile de qualité HPLC et on agite fortement à cause de la faible miscibilité de l'alcaloïde dans ce solvant. On ajoute par une ampoule à solide le Selectfluor™ (13,6 mmol, 1 équivalent) en 15 mn. Le mélange réactionnel se décolore complètement pour devenir limpide. On laisse agiter 2 h supplémentaires à température ambiante. Puis, on évapore l'acétonitrile à l'évaporateur rotatif et coévaporation avec de l'acétone : une mousse se forme dans le ballon à la fin de l'évaporation. On redissout cette mousse dans de l'acétone et l'on ajoute goutte à goutte une solution d'acétone contenant un équivalent d'H₂SO₄ concentré à 96% par une ampoule à brome au goutte à goutte : un précipité blanc se forme dans le mélange. On filtre la solution et on a alors le 1-chlorométhyl-4-aza-1-azoniabicyclo[2.2.2]octane tétrafluoroborate pur. On a alors le sulfate d'amine correspondant au produit de départ que l'on élimine. On ajoute environ 250 ml d'éther jusqu'à ce qu'il y ait formation d'un précipité blanc. Une filtration permet de récupérer le composé désiré sous forme d'un solide blanc avec un rendement de 98%. Le même mode opératoire a permis d'obtenir divers ammoniums selon la présente invention.

| Agent de Fluoration (tétrafluoroborate) | Rdt % |
|---|---|
| N-fluorocinchonidinium | 98 |
| N-fluorocinchoninium | 92 |
| N-fluoroquininium | 67 |
| N-fluoroquinidinium | 62 |

### Analyse du tétrafluoroborate de N-fluorocinchonidinium :

RMN¹H (300 MHz, CD₃CN): 9,08 (d, J = 5,4 Hz, 1 H); 8,80 (bs, 1 H); 8,30-7,95 (m, 5 H); 6,61 (s, 1 H); 5,78 (m, 1 H); 5,23 (s, 1 H); 5,16 (m, 1 H). 5,00 (m, 1 H); 4,48 (m, 2 H) ; 4,38 (m, 1 H) ; 3,45 (m, 2 H) ; 3,24 (m, 1 H). 2,62 (m, 2 H) ; 2,20 (m, 2 H) ; 2,19 (m, 1 H) ; 1,98 (m, 1 H).
RMN¹³C (75 MHz, CD₃CN): 153,31 ; 147,22 ; 141,62 ; 136,50 ; 134,36 ; 130,88 ; 125,93; 125,12; 124,40; 120,98; 118,49; 74,40 (d, J = 9,4 Hz); 68,58 (d, J = 9,2 Hz) ; 63,75 (d, J = 4,7 Hz) ; 59,62 (d, 9,2 Hz) ; 43,36 (d, J = 3,5 Hz) ; 28,58 (d, J = 4,7 Hz) ; 27,40 (d, J = 4,9 Hz) ; 23,90.
RMN¹⁹F (282 MHz, CD₃CN/CCl₃F) : 37,71 (s, 1 F); -154,52 (s, 1 F); -154,60 (s, 3 F). (CD₃CN/TFA) : 118,35 (s, 1 F); -74,12 (s, 1 F) ; -74,17 (s, 3 F).
IRFT : (ν N-F) 927 cm⁻¹
Point de fusion :189 +/-2°C
Solubilité : acétonitrile (ca 200 mg/ml), eau (très soluble), acétone (très peu soluble).
SM (FAB⁺, glycérol) : 313 (cation⁺, 18%); 295 (cation-H₂O, 100).
CCM : acétone/heptane : 9/1 ; Rf = 0.3.

### Exemple 2 - Synthèse de l'éther d'énol silylé de la 2-méthyl-1-tétralone

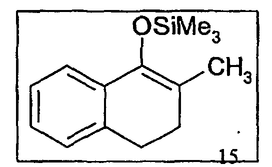

Dans un ballon de 5 ml, on place successivement la 2-méthyl-1-tétralone (0,75 g, 710 ml, 4,68 mmol, 1 équivalent), NEt₃ (0,592 g, 813 ml, 5,851 mmol, 1.2 équivalent), TMSCI (0,636 g, 743 ml, 5,851 mmol, 1,2 équivalent), on obtient une solution jaune limpide. On additionne lentement Nal (0,878 g, 5,851 mmol, 1,2 équivalent) en solution dans 6 ml d'acétonitrile : un précipité marron se forme. On agite pendant 4 h à température ambiante : on a alors un liquide brun avec beaucoup d'insolubles blancs en suspension. On filtre le mélange pour obtenir un solide blanc et un filtrat brun. Le filtrat est extrait avec trois fois 10 ml de pentane. La phase du pentane est jaune limpide très claire. Un flash chromatographie sur gel silice (éluant 90% heptane/10% éther) permet d'obtenir après évaporation des solvants, un liquide visqueux jaune pâle avec un rendement de 94% est obtenu.

### Analyse :

RMN¹H (300 MHz, CDCl₃): 7.40 (d, J = 7.4 Hz, 1 H) ; 7.26 (m, 1 H) ; 7.17 (t, J = 2.7 Hz, 2 H) ; 2.82 (t, J = 7.5 Hz, 2 H) ; 2.34 (t, J = 8 Hz, 2 H) ; 1.9 (s, 3 H); 0.29 (s, 9 H).
RMN¹³C (75 MHz, CD₃CN): 142.83 (Cq), 136.35 (CH), 134.78 (Cq), 127.13 (CH), 126.59 (Cq), 126.48 (CH), 121.91 (Cq), 117.35 (CH), 29.52 (CH₂), 28.68 (CH₂), 17.77 (CH₃), 1.02 (CH₃).

### Exemple 3 - Fluoration de la 2-méthyl-1-tétralone

### 1. Passage par son énolate

Dans un ballon de 5 ml, on place 2 équivalents de NaH à 95% (20,2 mg, 0,8 mmol) en solution à température ambiante sous atmosphère d'argon dans 1 ml de THF anhydre. La suspension est agitée 10 mn puis on ajoute la 2-méthyl-1-tétralone (0,33 mmol, 52,8 mg, 1 équivalent) goutte à goutte toujours à température ambiante, on voit alors que le mélange réactionnel change de coloration avec la formation de l'énolate. On abaisse la température du mélange réactionnel à -40°C et on laisse le ballon pendant 10 mn sous agitation. On additionne le tétrafluoroborate de N-fluorocinchonidinium (160 mg, 0,4 mmol, 1,2 équivalent) en solution dans 1 ml d'acétonitrile au pousse-seringue en 2 h : le mélange réactionnel change d'aspect. On arrête la réaction en hydrolysant avec 2 ml d'eau. Les produits sont extraits avec trois fois 10 ml d'éther puis séchés sur MgSO₄. La purification se fait par chromatographie sur gel de silice (éluant : heptane/éther : 9/1) conduisant à la 2-fluoro-2-méthyl-1-tétralone avec un rendement de 94%. L'excès énantiomérique mesuré en CPG est de 56%.

### 2. A partir de l'éther d'énol silylé

Dans un ballon de 5 ml, on place additionne le tétrafluoroborate de N-fluorocinchonidinium (89,6 mg, 0,224 mmol, 1 équivalent) en suspension dans 1 ml de THF et on abaisse la température à -78°C. On additionne l'éther d'énol silylé de la 2-méthyl-1-tétralone (40 mg, 46 µL) en solution dans 1 ml de THF en 2 h au pousse-seringue. Au bout de 24 h, on arrête la réaction en hydrolysant avec 2 ml d'HCI2N. Les produits sont extraits avec trois fois 10 ml d'éther puis séchés sur MgSO₄. La purification se fait par chromatographie sur gel de silice (éluant : heptane/éther : 9/1) conduisant à la 2-fluoro-2-méthyl-1-tétralone avec un rendement de 51%. L'excès énantiomérique mesuré en CPG est de 92%.

### Analyse :

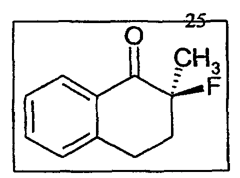

RMN¹H (300 MHz, CDCl₃): 8.00 (t, J = 2 Hz, 1 H) ; 7.40 (h, J = 2.5 Hz, 1 H) ; 7.21 (m, 2 H); 2.98 (m, 2 H) ; 2.20 (m, 2 H) ; 1.55 (d, J = 22 Hz, 3 H).
RMN¹⁹F (282 MHz, CDCl₃/TFA) :9.81 (1 F).
CCM : Et₂O/Heptane : 1/1 ; Rf = 0.4.
CPG Chirale : programmation de température : température initiale 65°C, 2 mn à 65°C, puis 5°/mn jusqu'à 170°C, 2-méthyl-1-tétralone : 20.70, 2-fluoro-2-méthyl-1-tétralone : 21.05 et 21.40.

### Exemple 4 - Fluoration de la 2,2,6-triméthylcyclohexanone

### Mode opératoire identique à 4-c-1.

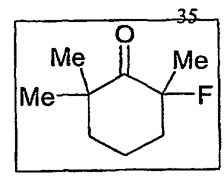

### Analyses :

CPG Chirale : programmation de température : température initiale 65°C, 2 mn à 65°C, puis 5°/mn jusqu'à 170°C, 2-fluoro-2,2,6 triméthylcyclohexanone : 7.50 et 7.75, 2,2,6 triméthylcyclohexanone : 8.69 et 8.95.

### Exemple 5 - Fluoration de la cyclopentanone-2-carboxylate d'éthyl

### Mode opératoire identique à 4-c-1.

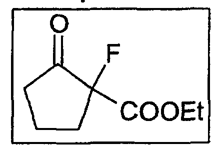

### Analyses :

RMN¹H (300 MHz, CDCl₃) : 4.33 (q, J = 7 Hz, 2 H) ; 2.55 (t, J = 7 Hz, 2 H) ; 2.34 (m, 2 H) ; 2.19 (q, J = 7.5 Hz, 2 H) ; 1.36(t,J=7Hz,3H).
RMN¹⁹F (282 MHz, CDCl₃/TFA) :-164.54 (t, 1 F).
CCM : Et₂O/Heptane : 1/1, Rf = 0.5.
CPG Chirale : programmation de température : température initiale 65°C, 2 mn à 65°C, puis 5°/mn jusqu'à 170°C, 2-fluorocyclopentanone-2-carboxylate d'éthyle :
14.70 et 14.85, cyclopentanone-2-carboxylate d'éthyle : 14.91.

### Exemple 6 fluoration d'alpha amino acide

Une solutionde N phtaloyl-2 phényl glycidonitrile (0,1 mmol, 26,24 mg) dans 2ml de THF refroidie à -78°C, on ajoute goutte à goutte une solution N dans Ir THF de Li HMDS (1,5 équiv., 0,15 mmol). Après 15 minute d'agitation dans les même conditions , on ajoute àlors en un seule fois 1,1 équivalent de l'agent de fluoration à savoir

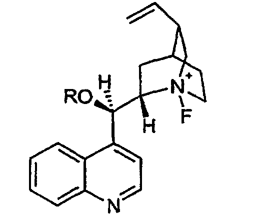

Avec R représentant paraméthoxybenzoyle.

La réaction est arrêtée après deux heures par addition de solution aqueuse saturée de chlorure d'ammonium. Après avoir chassé les solvants, le mélange est repris à l'éther et soumis à chromatographie préparative. On obtient un rendement de 93% environ et un excès énantiomérique de 94%.

## Revendications

1. Ammonium quaternaire présentant au moins un atome asymétrique dont la fonction ammonium quaternaire est porteuse d'un fluor et n'est distante dudit atome asymétrique par le chemin le plus court que d'au plus 4 chaînons, avantageusement au plus 3, de préférence au plus 2.

2. Ammonium selon la revendication 1, **caractérisé par le fait que** l'atome asymétrique est un atome de carbone.

3. Ammonium selon les revendications 1 et 2, **caractérisé par le fait que** l'atome asymétrique est un atome de carbone porteur d'une fonction hydrogénophore non oxydable.

4. Ammonium selon les revendications 1 à 3, **caractérisé par le fait que** l'atome asymétrique un atome de carbone porteur d'une fonction hydroxyle.

5. Ammonium selon les revendications 1 à 2, **caractérisé par le fait que** l'atome asymétrique un atome de carbone porteur d'une fonction hydrogénophore, avantageusement hydroxyle, protégée.

6. sous Ammonium selon les revendications 1, 2 et 5, **caractérisé par le fait que** l'atome asymétrique un atome de carbone porteur d'une fonction hydroxyle, protégée sous la forme d'un ether ou d'un ester.

7. Ammonium selon les revendications 1 à 6, **caractérisé par le fait que** l'atome asymétrique un atome de carbone porteur d'une un radical arylique ou aralcoylique.

8. Ammonium selon les revendications 1 à 7, **caractérisé par le fait que** l'azote de la fonction ammonium porteuse du fluor est intracyclique.

9. Ammonium selon les revendications 1 à 8, **caractérisé par le fait que** l'azote de la fonction ammonium porteuse du fluor est intracyclique à au moins deux cycles.

10. Ammonium selon les revendications 1 à 9, **caractérisé par le fait qu'**il présente un atome de carbone asymétrique relié à la fois à un radical porteur de la fonction ammonium fluorée, à un radical, distinct du précédent, porteur d'un noyau aromatique, et à une fonction hydrogénophore, avantageusement à une fonction alcool.

11. Ammonium selon les revendications 1 à 10, **caractérisé par le fait qu'**il est associé à un contre-ion non polarisant et mauvais nucléophile

12. Ammonium selon les revendications 1 à 11, **caractérisé par le fait qu'**il est associé à un contre-ion dont l'acide associé présente un pKa au plus égal à 2, avantageusement à 1, de préférence à 0.

13. Ammonium selon les revendications 1 à 12, **caractérisé par le fait qu'**il est associé à un contre-ion polyatomique.

14. Ammonium selon les revendications 1 à 13, **caractérisé par le fait qu'**il est associé à un contre-ion fluoré.

15. Réactif utile pour une fluoration électrophile, **caractérisé par le fait qu'**il comporte un ammonium quaternaire selon les revendications 1 à 14.

16. Réactif selon la revendication 15, **caractérisé par le fait qu'**il comporte en outre un solvant aprotique polaire.

17. Réactif selon les revendications 15 et 16, **caractérisé par le fait qu'**il comporte en outre pour addition successive ou simultanée une base.

18. Réactif selon les revendications 15 et 17, **caractérisé par le fait qu'**il comporte en outre, pour addition successive ou simultanée, une base dont l'acide associé présente un pKa au moins égal à 18.

19. Réactif selon les revendications 15 et 18, **caractérisé par le fait qu'**il comporte en outre, pour addition successive ou simultanée, une base engendrée in situ, avantageusement par un ion fluorure réagissant sur un alcool ou une amine silylée.

20. Synthèse des composés selon les revendications 1 à 12, **caractérisée par le fait qu'**ils consistent à mettre en contact dans un solvant polaire l'amine correspondante audit ammonium quaternaire avec un N-fluoro-1,4-diazoniabicyclooctane sous forme d'un sel dissocié.

21. Procédé de fluoration, **caractérisé par le fait qu'**il consiste à mettre en contact un réactif selon les revendications 13 à 17 avec un substrat riche en électrons.

22. Procédé selon la revendication 18, **caractérisé par le fait que** la réaction est menée à une température d'au plus 20°C.

23. Utilisation du réactif selon les revendications 13 à 17 pour une fluoration électrophile énantiosélective.

## Patentansprüche

1. Quaternäres Ammonium mit mindestens einem asymmetrischen Atom, dessen quaternäre Ammoniumfunktion von einem Fluor getragen wird und von dem genannten asymmetrischen Atom nur durch den kürzesten Weg von höchstens 4 Kettengliedern, vorteilhafterweise höchstens 3 Kettengliedern, vorzugsweise höchstens 2 Kettengliedern entfernt ist.

2. Ammonium nach Anspruch 1, **dadurch gekennzeichnet, daß** das asymmetrische Atom ein Kohlenstoffatom ist.

3. Ammonium nach Anspruch 1 und 2, **dadurch gekennzeichnet, daß** das asymmetrische Atom ein Kohlenstoffatom ist, das eine nicht oxidierbare, hydrogenophore Funktion trägt.

4. Ammonium nach Anspruch 1 bis 3, **dadurch gekennzeichnet, daß** das asymmetrische Atom ein Kohlenstoffatom ist, das eine Hydroxylfunktion trägt.

5. Ammonium nach Anspruch 1 bis 2, **dadurch gekennzeichnet, daß** das asymmetrische Atom ein Kohlenstoffatom ist, das eine hydrogenophore Funktion trägt, vorteilhafterweise geschütztes Hydroxyl.

6. Ammonium nach Anspruch 1, 2 und 5, **dadurch gekennzeichnet, daß** das asymmetrische Atom ein Kohlenstoffatom ist, das eine Hydroxylfunktion trägt, geschützt in Form eines Ethers oder eines Esters.

7. Ammonium nach Anspruch 1 bis 6, **dadurch gekennzeichnet, daß** das asymmetrische Atom ein Kohlenstoffatom ist, das einen arylischen oder aralkylischen Rest trägt.

8. Ammonium nach Anspruch 1 bis 7, **dadurch gekennzeichnet, daß** der Stickstoff der Ammoniumfunktion, der das Fluor trägt, intracyclisch ist.

9. Ammonium nach Anspruch 1 bis 8, **dadurch gekennzeichnet, daß** der Stickstoff der Ammoniumfunktion, der das Fluor trägt, intracyclisch bei mindestens zwei Ringen ist.

10. Ammonium nach Anspruch 1 bis 9, **dadurch gekennzeichnet, daß** es ein asymmetrisches Kohlenstoffatom aufweist, das sowohl mit einem Rest verbunden ist, der die fluorierte Ammoniumfunktion trägt, als auch mit einem Rest, vom vorstehenden verschieden, der einen aromatischen Kern und eine hydrogenophore Funktion trägt, vorteilhafterweise eine Alkoholfunktion.

11. Ammonium nach Anspruch 1 bis 10, **dadurch gekennzeichnet, daß** es mit einem nicht polarisierenden und schlechten nucleophilen Gegenion assoziiert ist.

12. Ammonium nach Anspruch 1 bis 11, **dadurch gekennzeichnet, daß** es mit einem Gegenion assoziiert ist, dessen assoziierte Säure einen pKa von höchstens gleich 2, vorteilhafterweise 1, vorzugsweise 0 aufweist.

13. Ammonium nach Anspruch 1 bis 12, **dadurch gekennzeichnet, daß** es mit einem polyatomischen Gegenion assoziiert ist.

14. Ammonium nach Anspruch 1 bis 13, **dadurch gekennzeichnet, daß** es mit einem fluorierten Gegenion assoziiert ist.

15. Reagens, geeignet für eine elektrophile Fluorierung, **dadurch gekennzeichnet, daß** es ein quaternäres Ammonium nach Anspruch 1 bis 14 umfaßt.

16. Reagens nach Anspruch 15, **dadurch gekennzeichnet, daß** es außerdem ein polares aprotisches Lösungsmittel umfaßt.

17. Reagens nach Anspruch 15 und 16, **dadurch gekennzeichnet, daß** es außerdem für die aufeinanderfolgende oder gleichzeitige Zugabe eine Base umfaßt.

18. Reagens nach Anspruch 15 und 17, **dadurch gekennzeichnet, daß** es außerdem für die aufeinanderfolgende oder gleichzeitige Zugabe eine Base umfaßt, deren assoziierte Säure einen pKa von mindestens gleich 18 aufweist.

19. Reagens nach Anspruch 15 und 18, **dadurch gekennzeichnet, daß** es außerdem für die aufeinanderfolgende oder gleichzeitige Zugabe eine Base umfaßt, die in situ erzeugt wird, vorteilhafterweise durch ein Fluoridion, das mit einem Alkohol oder einem silyliertem Amin reagiert.

20. Synthese der Verbindungen nach Anspruch 1 bis 12, **dadurch gekennzeichnet, daß** sie darin besteht, in einem polaren Lösungsmittel das Amin, das dem genannten quaternären Ammonium entspricht, mit einem N-Fluor-1,4-diazoniabicyclooctan in Form eines dissoziierten Salzes in Kontakt zu bringen.

21. Verfahren zur Fluorierung, **dadurch gekennzeichnet, daß** es darin besteht, ein Reagens nach Anspruch 13 bis 17 mit einem Substrat in Kontakt zu bringen, das reich an Elektronen ist.

22. Verfahren nach Anspruch 18, **dadurch gekennzeichnet, daß** die Reaktion bei einer Temperatur von höchstens 20 °C durchgeführt wird.

23. Verwendung des Reagens nach Anspruch 13 bis 17 für eine enantioselektive elektrophile Fluorierung.

## Claims

1. Quaternary ammonium compound having at least one asymmetric atom whose quaternary ammonium functional group bears a fluorine and is distant from said asymmetric atom by the shortest route by only at most 4 members, advantageously at most 3, preferably at most 2.

2. Ammonium compound according to Claim 1, **characterized in that** the asymmetric atom is a carbon atom.

3. Ammonium compound according to Claims 1 and 2, **characterized in that** the asymmetric atom is a carbon atom bearing a nonoxidizable hydrogenophore functional group.

4. Ammonium compound according to Claims 1 to 3, **characterized in that** the asymmetric atom is a carbon atom bearing a hydroxyl functional group.

5. Ammonium compound according to Claims 1 and 2, **characterized in that** the asymmetric atom is a carbon atom bearing a protected hydrogenophore, advantageously hydroxyl, functional group.

6. Ammonium compound according to Claims 1, 2 and 5, **characterized in that** the asymmetric atom is a carbon atom bearing a hydroxyl functional group, protected in the form of an ether or of an ester.

7. Ammonium compound according to Claims 1 to 6, **characterized in that** the asymmetric atom is a carbon atom bearing an aryl or aralkyl radical.

8. Ammonium compound according to Claims 1 to 7, **characterized in that** the nitrogen of the ammonium functional group bearing the fluorine is intracyclic.

9. Ammonium compound according to Claims 1 to 8, **characterized in that** the nitrogen of the ammonium functional group bearing the fluorine is intracyclic with at least two rings.

10. Ammonium compound according to Claims 1 to 9, **characterized in that** it has an asymmetric carbon atom linked simultaneously to a radical bearing the fluorinated ammonium functional group, to a radical, distinct from the preceding one, bearing an aromatic ring, and to a hydrogenophore functional group, advantageously to an alcohol functional group.

11. Ammonium compound according to Claims 1 to 10, **characterized in that** it is combined with a counter-ion which is nonpolarizing and a poor nucleophile.

12. Ammonium compound according to Claims 1 to 11, **characterized in that** it is combined with a counter-ion in which the associated acid has a pKa at most equal to 2, advantageously to 1, preferably to 0.

13. Ammonium compound according to Claims 1 to 12, **characterized in that** it is combined with a polyatomic counter-ion.

14. Ammonium compound according to Claims 1 to 13, **characterized in that** it is combined with a fluorinated counter-ion.

15. Reagent useful for electrophilic fluorination, **characterized in that** it comprises a quaternary ammonium compound according to Claims 1 to 14.

16. Reagent according to Claim 15, **characterized in that** it comprises, in addition, a polar aprotic solvent.

17. Reagent according to Claims 15 and 16, **characterized in that** it comprises, in addition, for successive or simultaneous addition, a base.

18. Reagent according to Claims 15 and 17, **characterized in that** it comprises, in addition, for successive or simultaneous addition, a base in which the associated acid has a pKa at least equal to 18.

19. Reagent according to Claims 15 and 18, **characterized in that** it comprises, in addition, for successive or simultaneous addition, a base generated in situ, advantageously by a fluoride ion reacting on an alcohol or a silylated amine.

20. Synthesis of the compounds according to Claims 1 to 12, **characterized in that** they consist in bringing the amine corresponding to said quaternary ammonium compound into contact, in a polar solvent, with an N-fluoro-1,4-diazoniabicyclooctane in the form of a dissociated salt.

21. Method of fluorination, **characterized in that** it consists in bringing a reagent according to Claims 13 to 17 into contact with a substrate rich in electrons.

22. Method according to Claim 18, **characterized in that** the reaction is carried out at a temperature of at most 20°C.

23. Use of the reagent according to Claims 13 to 17 for an enantioselective electrophilic fluorination.
